Europäisches Patentamt

European Patent Office

Office européen des brevets

(11) Publication number: 0 350 780
A1

# EUROPEAN PATENT APPLICATION

(21) Application number: 89112314.3

(51) Int. Cl.⁴: A61B 17/16

(22) Date of filing: 06.07.89

(30) Priority: 13.07.88 IT 1812188

(43) Date of publication of application:
17.01.90 Bulletin 90/03

(84) Designated Contracting States:
AT BE CH DE ES FR GB GR IT LI LU NL SE

(71) Applicant: Rosa, Mauro
Via Bellini 15
I-46010 Curtatone (Mantova)(IT)

Applicant: Venturi, Plinio
Via Arrivabene 61
I-46100 Mantova(IT)

(72) Inventor: Rosa, Mauro
Via Bellini 15
I-46010 Curtatone (Mantova)(IT)
Inventor: Venturi, Plinio
Via Arrivabene 61
I-46100 Mantova(IT)

(74) Representative: Modiano, Guido et al
MODIANO, JOSIF, PISANTY & STAUB
Modiano & Associati Via Meravigli, 16
I-20123 Milano(IT)

(54) Centering device for providing bone tunnels in femoral condyles.

(57) The present invention relates to a centering device for providing bone tunnels in femoral condyles, comprising a curved body (1) having, at one end thereof, a guide (2) for permitting sliding and locking of a slider (3) supporting a bush-holder (4) which contains a bush (5) for guiding a drill bit. The body (1) is shaped, at its other end, according to an S-shaped line which has the intrados (6) of the first curve directed toward the extrados of the curved body (1) and the intrados (7) of the second curve directed in the opposite direction; the second curve terminates at the end of the curved body which supports a head (9) in a position which faces the bush-holder (4) and is provided with teeth (9a) for ensuring stable contact with the femoral condyle and with a cavity for accommodating the drill bit.

Fig.1

# CENTERING DEVICE FOR PROVIDING BONE TUNNELS IN FEMORAL CONDYLES

The invention relates to a centering device for providing bone tunnels in femoral condyles.

It is known that during orthopedic surgery it is often necessary to perforate bones with drill bits to produce so-called bone tunnels.

That is what occurs, for example, in case of rupture of the knee's front or rear crossed ligament: in this case the surgeon must provide a tunnel in the femoral condyle wherethrough the new ligament, which may be an artificial or natural tendon, is passed.

In providing the tunnel, the surgeon must operate with extreme precision and must therefore be certain that the drill bit does not leave the preset optimum path; for this purpose, he uses a tool which has the function of guiding the bit during drilling, but said tool according to the known art, has some disadvantageous characteristics.

One of these disadvantageous characteristics resides for example in the fact that in order to place the tool in position it is necessary to force the patella to move from its position, thus creating an undesiderable condition; the obtainable securing of the tool is never stable enough to avoid requiring the action of one of the surgeon's assistants who must keep the tool in place throughout the required time.

Another disadvantageous characteristic of the known tools resides in the fact that the abutment element of the drill bit, intended to make contact with said bit at the end of the drilling of the tunnel, is pointed, and when the drill bit makes contact with said point it rapidly deteriorates, and thus requires continuous sharpening which, if not performed perfectly, may cause the incorrect penetration of said bit with vibration upon contacting the abutment element, with disastrous results for the tunnel being formed.

The aim of the present invention is to provide a centering device for providing bone tunnels in femoral condyles which can be placed with extreme rapidity and without having to move the contiguous organs and can be safely fixed so that it does not require the aid of securing performed manually by an operator.

Within the scope of the above described aim, an object of the invention is to provide a centering device which does not cause the rapid wear of the drill bits with which it interacts, with all the above described problems related to such wear.

The proposed aim and object, as well as other objects which will become apparent hereinafter, are achieved by a centering device for providing bone tunnels in femoral condyles, according to the invention, characterized in that it comprises a curved body having, at one end, a guide for a slider, with the possibility of locking said slider in a desired position, said slider supporting a bush-holder adapted to interchangeably contain a bush for the passage and guiding of a drill bit, said body being shaped, at its other end, along an S-shaped line in which the intrados of the first curve is directed toward the extrados of the curved body in order to accommodate the tibial plate, the intrados of the second curve being directed in the opposite direction in order to accommodate the patella, said second curve terminating at the end of said curved body which rotatably supports a head so that it faces said bush-holder, said head having means adapted to ensure stable contact with the surface of the femoral condyle and having a cavity for accommodating the drill bit.

Said head is advantageously connected to the end of the curved body by means of a thread and has front teeth adapted to make contact with the femoral condyle to ensure stability of said contact.

Further characteristics and advantages will become apparent from the description of a preferred but not exclusive embodiment of the invention, illustrated only by way of non-limitative example in the accompaning drawings, wherein:

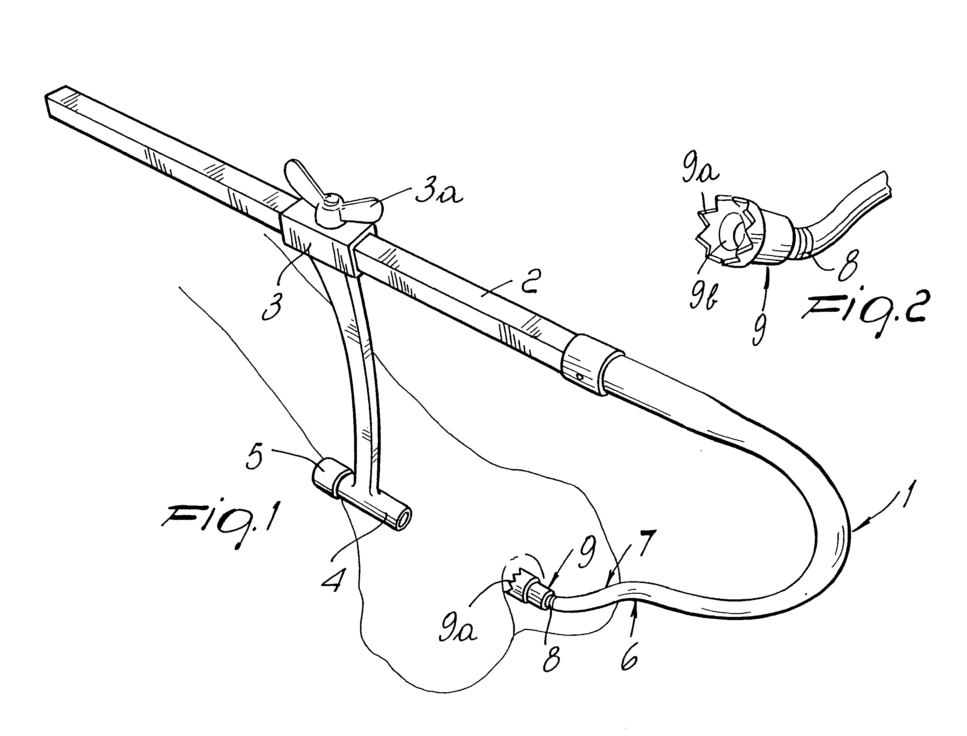

figure 1 is a perspective view of the invention in an operating position on a femoral condyle, illustrated in phantom lines only by way of reference;

figure 2 is a detail view of the head.

With reference to the above described figures, the reference numeral 1 indicates the curved body, which is provided, at one end, with the guide 2 on which the slider 3 may slide so as to be locked in the correct position by means of the butterfly screw 3a; said slider 3 supports the bush-holder 4 which is adapted to interchangeably contain a bush 5 through which a drill bit capable of providing the tunnel in the femoral condyle is adapted to pass so that it is guided.

The main characteristic of the invention resides in the fact that the curved body 1 is shaped, at its other end, like an "S" in which the intrados 6 of the first curve is directed toward the extrados of the curved body 1, so as to allow the accommodation of the tibial plate, and the intrados 7 of the second curve is directed in the opposite direction so as to accommodate the patella. The head 9 is associated with the end of the S-shaped portion by means of the threads 8; said head is arranged facing the bush 5 and is provided with front teeth 9a, adapted to make contact with the femoral condyle so as to ensure the stability of said contact, and with the cavity 9b for accommodating the drill bit.

From what has been described it is evident that the device according to the invention has characteristics of great functionality: its placement is in fact greatly simplified with respect to the known art, as the invention is appropriately placed without having to force the adjacent organs, differently from what occurs if one adopts the currently available device, which as mentioned entail a considerable movement of the patella; this circumstance, which is allowed both by the particular configuration of the device and by the adjustments allowed by the head 9, entails considerable rapidity in executing the operation and most of all allows the operator to have both hands free; said operator, after fixing the device, and without requiring a assistant to keep it in place as occurs with known devices, can provide the tunnel by passing the bit of a drill in the bush 5, with the assurance that said bit will follow the required path to the accommodating cavity 9b which ensures its integrity and is provided in the firmly fixed head 9.

The coupling of the head 9 to the body of the device by means of the thread 8 evidently allows, besides the described adjustment motions, easy and rapid replacement of said head when necessary.

The described invention is susceptible to numerous modifications and variations, all of which are within the scope of the inventive concept, and all the details may be replaced with other technically equivalent elements.

In the practical embodiment of the invention, the materials employed, as well as the shapes and dimensions, may be any according to the requirements.

Where technical features mentioned in any claim are followed by reference signs, those reference signs have been included for the sole purpose of increasing the intelligibility of the claims and accordingly such reference signs do not have any limiting effect on the scope of each element identified by way of example by such reference signs.

## Claims

1. Centering device for providing bone tunnels in femoral condyles, characterized in that it comprises a curved body having, at one end, a guide for a slider with the possibility of locking said slider in the desired position, said slider supporting a bush-holder adapted to interchangeably contain a bush for the passage and guiding of a drill bit, said curved body being shaped, at its other end, like an S with the intrados of the first curve directed toward the extrados of said curved body in order to accommodate the tibial plate, and with the intrados of the second curve directed in the opposite direction so as to accommodate the patella, said second curve terminating at the end of said curved body which rotatably supports a head so that it faces said bush-holder, said head having means adapted to ensure stable contact with the surface of the femoral condyle and having a cavity for accommodating the drill bit.

2. Device according to claim 1, characterized in that said head is connected to the end of the curved body by at least one thread.

3. Device according to one or more of the preceding claims, characterized in that said head has front teeth adapted to make contact with the femoral condyle in order to ensure the stability of said contact.

3a

2

3

5

Fig.1

4

9a

9

7

8

6

1

9a

9b

9

8

Fig.2

EP 0 350 780 A1

**EUROPEAN SEARCH REPORT**

Application Number

EP 89 11 2314

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | DE-A-2 747 568 (SCHMIDT-RAMSIN) * Page 7, line 11 - page 8, line 21; figure 1 * | 1,3 | A 61 B 17/16 |
| Y | US-A-4 257 411 (CHO) * Column 5, line 47 - column 6, line 16; column 6, line 67 - column 7, line 2; figure 1 * | 1,3 | |

TECHNICAL FIELDS SEARCHED (Int. Cl.5)

A 61 B

The present search report has been drawn up for all claims

| Place of search | Date of completion of the search | Examiner |
|---|---|---|
| THE HAGUE | 02-10-1989 | MOERS R.J. |

EPO FORM 1503 03.82 (P0401)